# EUROPÄISCHE PATENTSCHRIFT

(11) **EP 1 196 439 B1**
(45) Veröffentlichungstag und Bekanntmachung des Hinweises auf die Patenterteilung: **03.08.2005**
(21) Anmeldenummer: 00931131.7
(22) Anmeldetag: 06.05.2000
(51) Int. Cl.: C07K 14/435, A61K 38/17, C12N 15/12, C12N 15/63, C12N 5/10, A61P 31/04

(54) **VERBINDUNGEN MIT ANTIBIOTISCHER WIRKUNG**
COMPOUNDS EXHIBITING AN ANTIBIOTIC ACTIVITY
COMPOSES A ACTION ANTIBIOTIQUE

(30) Priorität: 07.05.1999 DE 19921027
(43) Veröffentlichungstag der Anmeldung: 17.04.2002
(73) Patentinhaber: BRAIN Biotechnology Research and Information Network Aktiengesellschaft, 64673 Zwingenberg (DE)
(72) Erfinder: REETZ, Manfred, D-45470 Mülheim a. d. Ruhr (DE); KÜHLING, Klaus, D-45470 Mülheim a. d. Ruhr (DE); MEHLHORN, Heinz, Heinrich-Heine-Univ. Düsseldorf, D-40225 Düsseldorf (DE); JÄGER, Karl-Erich, Ruhr Univ. Bochum, D-44790 Bochum (DE)
(74) Vertreter: VOSSIUS & PARTNER
(86) Internationale Anmeldenummer: PCT/EP2000/004089
(87) Internationale Veröffentlichungsnummer: WO 2000/068258

(56) Entgegenhaltungen:
- K. OBARA ET AL.: "Molecular cloning of the antibacterial protein of the giant African snail, Achatina fulica Ferussac" EUROPEAN JOURNAL OF BIOCHEMISTRY, Bd. 209, Nr. 1, Oktober 1992 (1992-10), Seiten 1-6, XP000960391
- N. TAKAMATSU ET AL.: "Molecular cloning of the defense factor in the albumen gland of the sea hare Aplysia kurodai" FEBS LETTERS, Bd. 377, Nr. 3, Dezember 1995 (1995-12), Seiten 373-376, XP002152015 AMSTERDAM NL
- DATABASE BIOSIS [Online] BIOSCIENCES INFORMATION SERVICE, PHILADELPHIA, PA, US; 1992 OTSUKA-FUCHINO H ET AL: "BACTERICIDAL ACTION OF A GLYCOPROTEIN FROM THE BODY SURFACE MUCUS OF GIANT AFRICAN SNAIL" Database accession no. PREV199294040663 XP002152016 & COMPARATIVE BIOCHEMISTRY AND PHYSIOLOGY C COMPARATIVE PHARMACOLOGY AND, Bd. 101, Nr. 3, 1992, Seiten 607-613, 1992 ISSN: 0742-8413

## Beschreibung

Die Erfindung betrifft Verbindungen mit antibiotischer Wirkung, die aus Körpersäften von Mollusken, nämlich von bestimmten westafrikanischen Schnecken, gewonnen werden, Arzneimittel, umfassend diese Verbindungen, sowie deren Verwendung zur Herstellung eines Medikaments zur Bekämpfung infektiöser Erreger bei Mensch und Tier.

### Hintergrund der Erfindung

Eine generelle Strategie zur Bekämpfung von infektiösen Erregern bei Mensch und Tier besteht in der Anwendung von Antibiotika, von denen bislang mehrere tausend verschiedene Verbindungen isoliert und verwendet wurden. Deren antibiotische Wirkung ist jedoch im Regelfall auf einige wenige nahe verwandte Erregerarten beschränkt. Ein zunehmend größer werdendes Problem beim Gebrauch von Antibiotika ist zudem das Auftreten resistenter Bakterien [J. Davies, *Science* 1994, *264*, 375-382. Inactivation of antibiotics and the dissemination of resistance genes]. Die Ausbreitung resistenter Bakterien dauert im Regelfall 2-5 Jahre, so dass ein ständiger Bedarf besteht, neue und effektive antibakteriell wirksame Mittel zu finden oder zu entwickeln. Hierbei wird versucht, auch sogenannte natürliche Antibiotika zu gewinnen und einzusetzen; das sind Peptide oder Proteine, die aus solchen Organismen gewonnen werden können, die mit Bakterien in Kontakt kommen, also aus Pflanzen, niedrigen und höheren Tieren und Menschen. Obwohl einige natürliche Antibiotika isoliert und charakterisiert worden sind, ist es bisher nicht gelungen, diese bis zum klinischen Einsatz fortzuentwickeln [J. E. Gabay, *Science* 1994, *264,* 373-374. Ubiquitous natural antibiotics].

Es wurde nun überraschenderweise gefunden, daß aus bestimmten westafrikanischen Schneckenarten, nämlich den Schnecken der Gattungen *Archachatina* (insbesondere der Arten *A. marginata, A. degneri,* und *A. ventricosa*) sowie von Vertretern der Gattung *Achatina* mit den Arten *A. achatina, A. monochromatica*, und *A. batteata*, Verbindungen mit antibiotischer Wirkung gewonnen werden können. U. a. kann ein Protein, das aus dem gelblichen Schleim der Fußsohlen dieser Schnecken isoliert und identifiziert wurde, gegen ein breites Spektrum infektiöser Erreger eingesetzt werden.

### Zusammenfassung der Erfindung

Die vorliegende Erfindung betrifft somit
(1) Verbindungen mit antibiotischer Wirkung, die aus Körpersäften von Schnecken der Gattung *Archachatina* und/oder der Arten *Achatina achatina, Achatina monochromatica* und *Achatina batteata* der Gattung *Achatina* isolierbar sind;
(2) in einer bevorzugten Ausführungsform sind die Verbindungen (1) ein Peptid, Protein oder Derivat derselben;
(3) eine DNA-Sequenz, die für ein Peptid, Protein oder Derivat derselben gemäß (2) codiert;
(4) einen Vektor, der eine DNA-Sequenz gemäß (3) umfaßt;
(5) einen Wirtsorganismus, der mit einem Vektor gemäß (4) transformiert ist und/oder der eine DNA-Sequenz gemäß (3) aufweist;
(6) ein Verfahren zur Herstellung des Peptids, Proteins oder des Derivats derselben nach (2), umfassend das Kultivieren eines Wirtsorganismus gemäß (5) und Isolieren des Peptids, Proteins oder Derivats derselben;
(7) Arzneimittel, enthaltend eine Verbindung nach (1) oder (2) und gegebenenfalls ein pharmazeutisch akzeptables Trägermaterial;
(8) Verwendung einer Verbindung nach (1) oder (2) zur Herstellung eines Medikaments zur Bekämpfung von infektösen Erregern bei Mensch und Tier;
(9) Nahrungs- oder Futtermittel, enthaltend eine Verbindung gemäß (1) oder (2) und
(10) ein Verfahren zur Behandlung infektiöser Erreger bei Mensch und Tier umfassend das Verabreichen einer Verbindung gemäß (1) oder (2).

### Figurenbeschreibung

Fig. 1: Molekulargewichtsbestimmung der antibiotisch wirksamen Substanz mit Polyacrylamid Gelelektrophorese (SDS-PAGE). Neben dem Größenstandard (M) in Spur 1 sind die einzelnen Fraktionen der Ionenaustauschchromatographie in den Spuren 2 bis 10 aufgetragen. Man erkennt deutlich, daß zuerst ein höhermolekulares Protein eluiert (Fr. 1 und 2), gefolgt von der antibakteriell wirksamen Substanz (Fr. 3 bis 6), die in Fraktion 4 in reiner Form und in höchster Konzentration vorkommt.
Fig. 2: Vergleich der Aminosäuresequenzen von fünf Peptiden aus dem antibakteriell wirksamen Protein aus *Archachatina marginata* mit dem antibakteriell wirksamen Protein Achacin aus *Achatina fulica*. Die Konsensus-Sequenzen sind zwischen den Aminosäuresequenzen von Achacin und den Peptiden angegeben; identische Aminosäuren sind im 1-Buchstaben-Code angegeben, ähnliche mit einem Pluszeichen, unterschiedliche mit einem Punkt markiert.
Fig. 3: Schnecke der Gattung *Archachatina,* hier *Archachatina marginata*.
Fig. 4: Eier von *Archachatina marginata*.

### Ausführliche Beschreibung der Erfindung

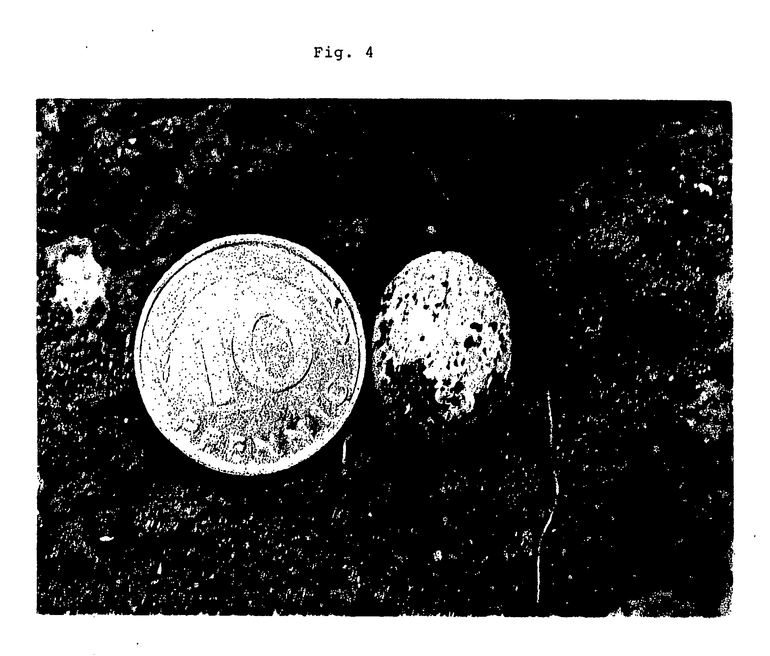

Die antibiotisch wirksamen Substanzen werden produziert von Schnecken der *Art Archachatina marginata*. Die Verbreitung dieser bis zu 15 cm großen Landschnecken in Westafrika wurde von Hodashi beschrieben [J. K. M. Hodashi, *Revue mondiale de Zootechnie* 1984, *52,* 24-35. Les escargots géants comestibles d'Afrique occidentale]. Äußerlich können die Schalen der Schnecken der Gattungen *Archachatina* und *Achatina* durch die Form der Schalenspitze unterschieden werden, wobei *Achatina* eine spitze, *Archachatina* aber eine stumpfe Spitze besitzt. Ein weiteres Unterscheidungsmerkmal ist die Größe der Eier: *Achatina* legt viele etwa 3-4 mm große, *Archachatina* legt wenige, etwa 1 cm große Eier (siehe Fig. 4). Die Schnecken leben vegetarisch und können im Labor nachgezüchtet werden, was ihre Verfügbarkeit gewährleistet.

Wirkstoffe, die aus diesen Schnecken gewonnen werden können, sind:
1. gelblicher Schleim aus/von der Fußsohle, zu gewinnen durch Abstreichen mit dem Skalpell oder durch Reizung mit Stromstößen von 0,5 - 1 mA,
2. weißlich bis bläuliches Wehrsekret, zu gewinnen nach Setzen einer kleinen Verletzung durch ein Skalpell; die Schnecke entläßt dieses Sekret in dickem Strahl aus der Mantelhöhle,
3. rötliche Hämolymphe, die nach Verletzung aus der Leibeshöhle austritt,
4. Schalenpulver, zu gewinnen durch Zermahlen der Schale.

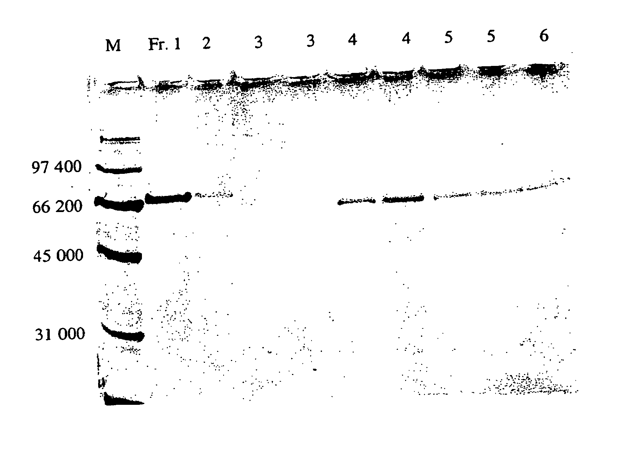

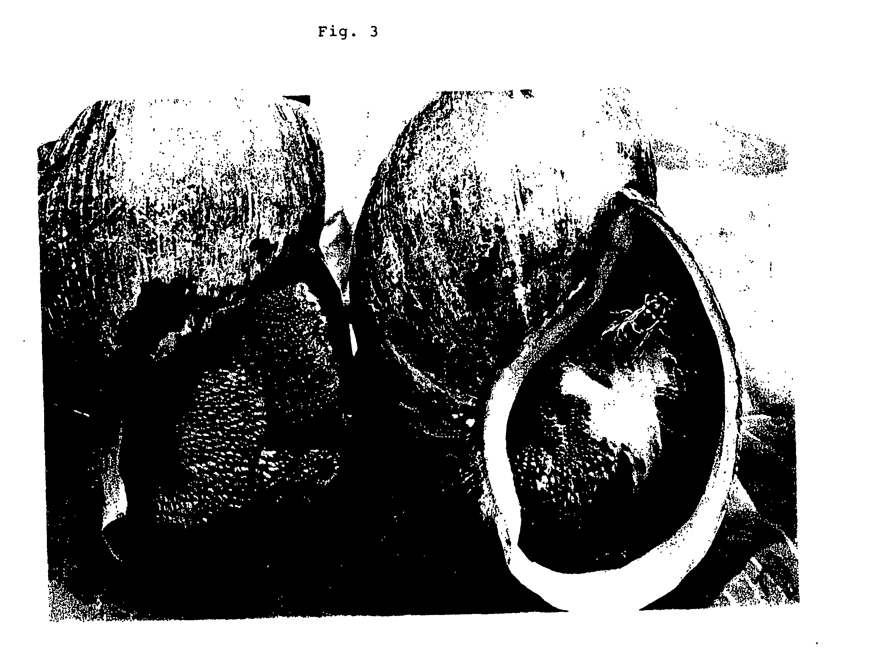

Am wirksamsten im Sinne der Erfindung ist ein Peptid, Protein oder Derivat derselben, das Bestandteil des gelblichen Schleims aus der Fußsohle der genannten Schnecke ist und insbesondere aus *Archachatina marginata* stammt (siehe Fig. 3). Die säulenchromatographische Aufreinigung mittels folgender Verfahrensschritte
(a) mehrfache Ultrafiltration;
(b) Gelpermeationschromatographie; und
(c) Ionenaustauscherchromatographie
bis zur elektrophoretischen Homogenität lieferte eine Fraktion, die in der SDS-PAGE eine Bande zeigte (Fig. 1), deren Intensität mit der biologischen Wirksamkeit korrelierte. Die antibakteriell wirksame Verbindung ist daher vorzugsweise ein Protein oder Proteinderivat mit einem apparenten Molekulargewicht von ca. 60 kDa. Dieses Ergebnis steht im Einklang mit den Ergebnissen, die bei der Ultrafiltration der wirksamen Verbindung erhalten wurden, nämlich daß das Molekulargewicht der gesuchten Substanz größer als 50 kDa sein muß.

Das Protein oder Proteinderivat im Sinne der vorliegenden Erfindung weist vorzugsweise mindestens eine, insbesondere alle acht der in Tab. 1 gezeigten Teilsequenzen auf.

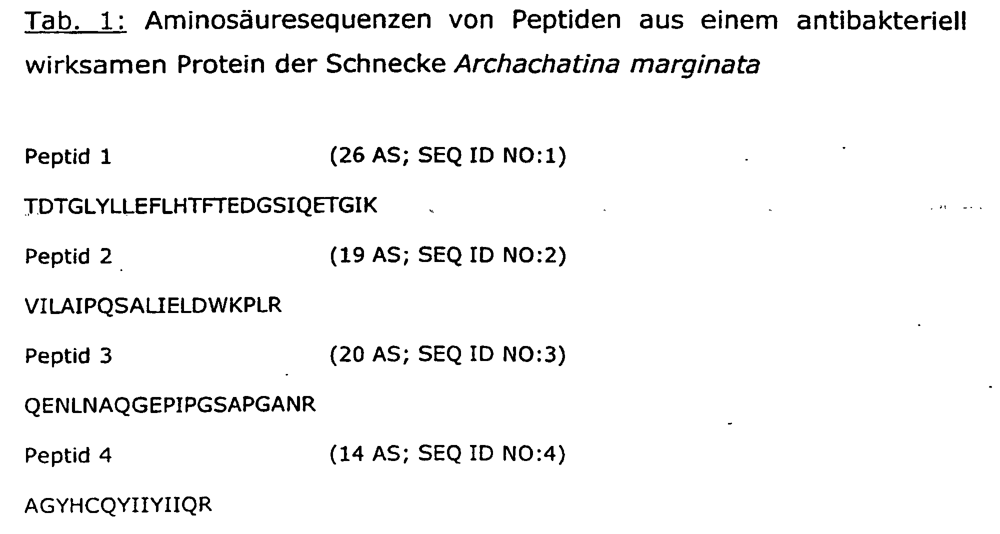

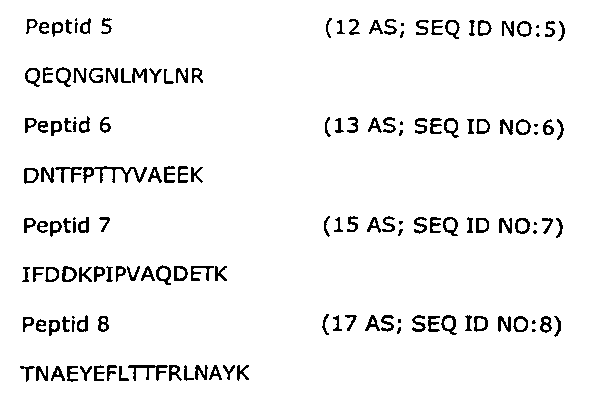

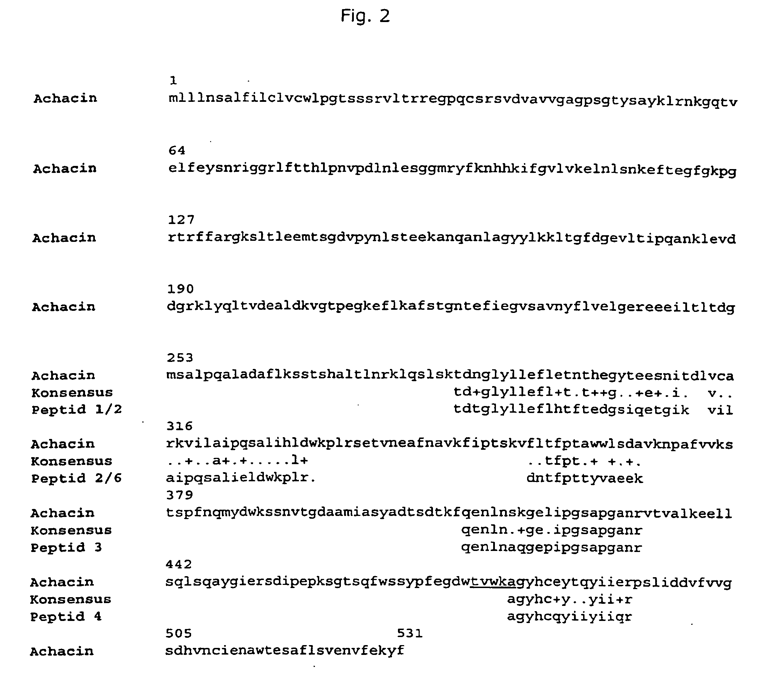

Die in Tab. 1 gezeigten Teilsequenzen von acht Peptiden aus dem wirksamen Protein wurden mit den in den zugänglichen Proteindatenbanken (SwissProt, EMBL, NCBI) vorhandenen Aminosäuresequenzen verglichen. Es konnte nur eine geringfügige Ähnlichkeit mit dem Protein Achacin aus der Schnecke *Achatina fulica* Férussac (Obara, 1992; Accession-Number: P35903) festgestellt werden, nämlich daß im Mittel nur 35 % der verglichenen Aminosäuren beider Proteine identisch sind (siehe Fig. 2). Von 136 bisher sequenzierten Aminosäuren sind 88 unterschiedlich, wie aus der nachfolgenden Tabelle 2 ersichtlich ist.

**Tab. 2:**

| Schnecken-peptide | Länge der Sequenz [AS] | untersch. AS^{1.)} (einschl. ähnliche) | Identität^{2.)}[%] |
|---|---|---|---|
| Peptid 1 | 26 | 11 | 58 |
| Peptid 2 | 19 | 16 | 16 |
| Peptid 3 | 20 | 3 | 85 |
| Peptid 4 | 14 | 4 | 71 |
| Peptid 5 | 12 | 13 | - |
| Peptid 6 | 13 | 9 | 31 |
| Peptid 7 | 15 | 15 | - |
| Peptid 8 | 17 | 17 | - |
| GESAMT: | 136 | 88 | 35 |

| | | | |
|---|---|---|---|
| ^{1.)} unterschiedliche Aminosäuren umfassen konservative Substitutionen (z. B. E/H; E/Q; S/T; V/P) | | | |
| ^{2.)} [%] Aminosäuren, die identisch mit aus *Achatina fulica* isoliertem Achacin sind | | | |

Weitere Homologien zu bisher bekannten Proteinen wurden nicht gefunden. Die antibiotisch wirksamen Verbindungen der vorliegenden Erfindung sind somit nicht identisch mit den in der Literatur beschriebenen Substanzen aus der Landschnecke *Achatina fulica* aus Okinawa (als Achacin bezeichnet) [H. Otsuka-Fuchino, Y. Watanabe, C. Hirakawa, T. Tamiya, J. J. Matsumoto, T. Tsuchiya, *Comp. Biochem. Physiol.* 1992, *101C(3),* 607-613. Bactericidal action of a glycoprotein from the body surface mucus of giant African snail; Y. Kubota, Y. Watanabe, H. Otsuka, T. Tamiya, T. Tsuchiya, J. J. Matsumoto, *Comp. Biochem. Physiol.* 1985, *82,* 345-348. Purification and Characterization of an Antibacterial Factor from Snail Mucus; K. Obara, H. Otsuka-Fuchino, N. Sattayasai, Y. Nonomura, T. Tsuchiya, T. Tamiya, *Eur. J. Biochem.* 1992, *209,* 1-6. Molecular cloning of the antibacterial protein of the giant African snail, *Achatina fulica* Férussac], sowie aus der Wasserschnecke aus Japan *Aplysia kurodai -* als Aplysienin-A bezeichnet [N. Takamatsu, T. Shiba, K. Muramoto, H. Kamiya, *FEBS Letters* 1995, *377,* 373-376. Molecular cloning of the defense factor in the albumen gland of the sea hare *Aplysia kurodai*.]

Der die wirksame Verbindung (d. h. das Peptid, Protein oder Derivat desselben) enthaltende Schleim ist bei einer Lagerung von -80 °C bis hin zu +40 °C stabil und verliert nicht seine pharmakologische bzw. antibakterielle Wirksamkeit. Ebenso wurde die antibakterielle Wirksamkeit durch Lagerung bei Raumtemperatur bei pH 8 bzw. pH 9,5 nicht beeinträchtigt. Bei Erhitzen über 70 °C bzw. 110 °C für 30 min geht die Wirksamkeit vollständig verloren. Sowohl die gereinigte Substanz als auch der isolierte Roh-Schleim lassen sich lyophilisieren und wieder in Wasser lösen, ohne die oben beschriebene Wirksamkeit einzubüßen. Auf diese Weise um das Fünffache aufkonzentrierte Fraktionen zeigten Hemmhöfe mit einem Durchmesser von bis zu 1,6 cm. Löslichkeitsuntersuchungen ergaben, daß sich die gesuchte Substanz in Wasser, nicht jedoch in Methanol, Hexan oder einem Chloroform/Methanol-Gemisch (4 : 1) löst.

Als wirksame Substanzen kommen neben den aus den obengenannten Schnecken direkt erhaltenen Substanzen in ungereingter oder gereinigter Form auch alle Arten von modifizierten Varianten, die z. B. bei Proteinen durch chemische Modifizierung von Aminosäuren einschließlich Glykolysierung, Ribosylierung, Acylierung, Phosphorylierung und anderen erhalten wurden, in Frage. Wirksam sind auch solche modifizierte Varianten, die durch gentechnische Methoden gewonnen werden. Hierzu gehören auch solche modifizierten Varianten, die dadurch erhältlich sind, dass DNA-Moleküle, die für die beschriebene Substanz kodieren und einer Mutagenese mit einer molekularbiologischen Technik (z. B. chemischer, gerichteter oder zufälliger Mutagenese, auch mit Methoden der PCR, Methoden der gerichteten Evolution, staggered extension process, DNA-shuffling u. a. [M. T. Reetz, K.-E. Jaeger, *Top. Curr. Chem*. 1999, *200* (Biocatalysis), 31-57. Superior biocatalysts by directed evolution]) unterworfen wurden, in den nachfolgend beschriebenen rekombinanten Techniken eingesetzt werden.

In der Regel werden die oben beschriebenen Substanzen in verschiedenen Verfahren aufbereitet, hergestellt und/oder gereinigt, die der jeweiligen Anwendung angemessen sind.

Die vorliegende Erfindung betrifft ebenfalls DNA-Sequenzen, die für die vorstehend definierten Peptide, Proteine und Derivate derselben (einschließlich Teilsequenzen, wie die in Tabelle 1 gezeigten) codieren. Diese DNA-Sequenzen können in geeignete Expressionsvektoren eingebracht werden und ein geeigneter Wirtsorganismus mit diesen Vektoren transformiert werden bzw. Wirtsorganismen mit den genannten DNA-Sequenzen transfiziert werden. Die transformierten/transfizierten Wirtsorganismen können dann zur Herstellung des gewünschten Peptids, Proteins oder Derivats verwendet werden. Geeignete Wirtsorganismen im Sinne der vorliegenden Erfindung sind Mikroorganismen, wie Bakterien (z. B. *E. coli*,) und Hefen und Säugerzellsysteme wie CHO-Zellen und immortalisierte Säugerzellen.

Die antibiotisch wirksamen Verbindungen gemäß der vorliegenden Erfindung können sowohl bei äußerlicher wie bei innerlicher Applikation Bakterien, Parasiten, Viren und andere infektiöse Erreger abtöten. Die infektiösen Erreger, gegen die die erfindungsgemäßen Verbindungen wirksam sind, sind solche, die Magen-Darm-Symptome hervorrufen, die über das Blut innere Organe befallen, und die zu offenen Wunden in der Haut führen.

So konnte die antibakterielle Wirksamkeit des aus *Archachatina marginata* isolierten Proteins mit einem apparenten Molekulargewicht von ca. 60kDa mit einem Bioassay gezeigt werden, wobei Bakterienisolate verwendet wurden, die frisch aus infizierten und hospitalisierten Patienten isoliert wurden. Eine antibakteriell-bakterizide Wirkung wurde unter anderem festgestellt gegen die Gram-negativen Species *Escherichia coli* und *Pseudomonas aeruginosa* und die Grampositiven Species *Staphylococcus aureus* und *Staphylococcus epidermidis* (siehe Beispiele 3 und 4).

Das vorstehend genannte Protein ist auch wirksam gegen andere infektiöse Erreger. Als Beispiel seien Malaria-Erreger der Gattung *Plasmodium* (verschiedene Arten) erwähnt. In dem in Beispiel 5 beschriebenen Testsystem führte die Verabreichung der Substanz an Malaria-infizierte Mäuse zu einer signifikant erhöhten Überlebensrate im Vergleich mit einer unbehandelten Kontrollgruppe.

Das erfindungsgemäße Arzneimittel kann in der folgenden Applikationsformen vorliegen:
a) oral als Lösung (sterilfiltriert und eventuell mit Wasser verdünnt), z. B. zur Kurierung von Infektionen des Magendarmtraktes (z. B. Gastritis),
b) Aufbringung als Lösung (wie a)) in offene Wunden zur Wundheilung bzw. Abtötung von Bakterien oder anderen Erregern in der Blutbahn (z. B. zur Verhinderung einer Sepsis),
c) Verabreichung isolierter, gereinigter und eventuell formulierter Fraktionen in der Form und mit Zielen von a) und b),
d) Verabreichung in der Form wie c), aber als Lyophilisat (z. B. zur Aufbringung auf offene Wunden zur Wundheilung).

Gegenstand der Erfindung sind auch Arzneimittel, die die erfindungsgemäßen Substanzen enthalten und zur Bekämpfung der Bakteriosen und anderen infektiösen Erregern bei Mensch und Tier eingesetzt werden können.

Arzneimittel zur oralen Anwendung können Pulver, Granulate, Emulsions- oder Suspensionskonzentrate sein, die allein oder mit der Nahrung vermischt Menschen oder Tieren verabreicht werden können. Derartige Arzneimittel lassen sich analog üblicher Verfahren herstellen, beispielsweise, indem der Wirkstoff (in Gänze oder in Fraktionen) mit festen oder flüssigen Trägerstoffen und eventuell unter Zugabe von Zusatzstoffen wie Arzneimittelhilfsstoffe, Emulgier- oder Dispergiermitteln, Lösungsmitteln, Farbstoffen, Konservierungsstoffen und/oder Antioxidantien vermischt wird. Geeignete Lösungsmittel sind dabei z. B. destilliertes Wasser und physiologische Salzlösung, geeignete Konservierungsstoffe sind z. B. Benzylalkohol, Benzalkoniumchloride und Phenol und geeignete Arzneimittelhilfsstoffe sind z. B. Carboxymethylcellulose und Natriumalginat.

Für Arzneimittel zur äußerlichen Anwendung gilt sinngemäß das gleiche. Auch bei Anwendung von wirksubstanzhaltigen Mitteln in der Blutbahn (intravenöser Anwendung) gilt das oben Gesagte.

Aufgrund der hohen Wirksamkeit der vorliegenden Verbindungen (Wirkung bereits im Femtomolbereich) genügen bereits geringe Mengen der Verbindung zum Erzielen des gewünschten therapeutischen Effekts. Die tägliche Dosis der erfindungsgemäßen Verbindung ist allerdings abhängig von der therapeutischen Aufgabenstellung, der körperlichen Konstitution des Patienten und der Applikationsform. So liegt die tägliche Dosis bei oraler und äußerlicher Anwendung im Bereich von 0.01 mg bis 10 g, vorzugsweise 1 mg bis 1 g, und bei intravenöser Anwendung im Bereich von 1 µg bis 10 g, vorzugsweise 0.1 mg bis 1 g.

Gegenstand der Erfindung sind auch Nahrungs- und Futtermittelzusätze, die die reinen oder fraktionierten Substanzen aus Schnecken der Gattungen *Achatina* und *Archachatina* oder aus reproduktiven Systemen enthalten und in geeigneter Weise den Nahrungsmitteln bzw. mit dem speziellen Futter von verschiedenen Tierarten vermischt werden. Die optimale wirksame Dosis zur Behandlung von Infektionen mit den genannten Bakterien oder infektiösen Erregern hängt von der Bakterien- bzw. Erregerart, der Art und Dauer der Behandlung, sowie vom Alter und Zustand der behandelten Menschen und Tiere ab.

Das Verfahren zur Behandlung der obengenannten Symptome bei Mensch und Tier schließt ein Verfahren zur Behandlung von Malaria beim Menschen ein.

Die vorliegende Erfindung wird anhand der nachfolgenden Beispiele näher erläutert.

### Beispiele

### Beispiel 1: Isolierung und Reinigung

Der aus der Fußsohle der Schnecke *Archachatina marginata* isolierte gelbe Schleim wurde durch mehrere Ultrafiltrationsschritte gereinigt. Nach Vorfiltration durch eine Membran der Porengröße 0,65 µm wurde das Filtrat auf eine Membran mit der nächst geringeren Ausschlußgrenze aufgebracht. Verwendet wurden Membranen der Firma Millipore/Amicon mit folgenden Ausschlußgrenzen (kDa): 100, 50, 30, 10 und 5. Wahlweise kann eine Zentrifugation für 1 h bei 31 000 x g (Zentrifuge Sorvall R2/5B, Rotor SS34, 20 000 rpm) erfolgen, wobei die im Schleim enthaltenen festen Bestandteile ein Pellet bilden. Der Überstand wurde mit einem Volumenäquivalent Pufferlösung (10 mM Tris / 100 mM NaCl, pH 8) verdünnt und durch Gelpermeationschromatographie (GPC) und anschließende Ionenaustauschchromatogaphie aufgereinigt. Als Trennsäule für die präparative GPC-Trennung wurde eine Fractogel EMD BioSEC Säule (Länge: 600 mm, Innendurchmesser: 15 mm, Merck) verwendet. Der Elutionspuffer bestand aus 10 mM Tris / 100 mM NaCl, pH 8; eluiert wurde mit einer Flußgeschwindigkeit von 1 ml/min bei einem Druck von 2,0 MPa. Die Detektion erfolgte spektralphotometrisch bei 210 nm. Für die ionenchromatographische Trennung der aufkonzentrierten GPC-Fraktionen wurde eine UNO Q6-Säule (Länge: 53 mm; Innendurchmesser: 12 mm, BioRad) verwendet. Der Elutionspuffer bestand aus 10 mM Tris / 100 mM NaCl, pH 9,5; eluiert wurde mit einer Flußgeschwindigkeit von 2 ml/min bei einem Druck von 3,8 MPa. Die Detektion erfolgte spektralphotometrisch bei 210 nm. Die Reinheit der erhaltenen Fraktionen wurde mit SDS-Polyacrylamid Gelelektrophorese (SDS-PAGE) und anschließender Silberfärbung der Gele überprüft.

### Beispiel 2: Bestimmung der Aminosäuresequenzen tryptischer Peptide

Die Peakfraktionen aus der säulenchromatographischen Trennung mit der höchsten biologischen Aktivität wurden mittels Ultrafiltration über Centricon 30 (Amicon) konzentriert. Das Protein wurde mit 5 M Guanidinium-hydrochlorid versetzt, über Nacht carboxymethyliert und mittels einer Mikrodialyseapparatur (Pierce) dialysiert. Das carboxymethylierte Protein (12 µg) wurde über Nacht tryptisch verdaut (Verhältnis 1 Teil Protein : 5 Teile Trypsin). Die Peptide wurden mittels HPLC auf einer reversed-phase Säule (C-18, 300 µm, Länge: 25 cm) aufgetrennt. Der Proteingehalt der Fraktionen wurde spektralphotometrisch bei 295 und 215 nm detektiert und von ausgewählten Fraktionen ein MALDI Massenspektrum aufgenommen. Nach Auswertung der Spektren wurden 8 Peptide ausgewählt und einer high-sensitive-Mikrosequenzierung auf einem ProSize 494 Sequencer (Perkin Eimer - Applied Biosystems) unterzogen. Die Sequenzen der Peptide sind vorstehend in Tabelle 1 zusammengefaßt.

### Beispiel 3: Bestimmung der antimikrobiellen Wirkung I

Die antibakterielle Aktivität der in Beispiel 1 erhaltenen Fraktionen wurde durch einen Bioassay untersucht. Hierzu wurde eine Kultur des humanpathogenen Bakteriums *Staphylococcus aureus* in Vollmedium (NB, Difco) für 3-4 h bei 37 °C unter Schütteln inkubiert bis die Bakterien eine Zelldichte von ca. 5 x 10⁸ ml⁻¹ (logarithmische Wuchsphase) erreicht hatten. Je 100 µl dieser Kultur wurden durch Ausspateln auf NB-Agarplatten gebracht, für ca. 30 min bei Raumtemperatur belassen und anschließend aliquote Teile der Fraktionen (Volumen: 5 oder 10 µl) auf die Platte pipettiert. Die Platten wurden über Nacht bei 37 °C inkubiert und die biologische Wirksamkeit durch Bestimmung des Durchmessers der auf dem Bakterienrasen gebildeten Hemmhöfe beurteilt, welcher bei den wirksamen Fraktionen ca. 10 - 16 mm betrug. Die Ergebnisse sind in Tabelle 3 zusammengefaßt.

**Tab. 3:**

| Bakterienstamm¹ | Klassifizierung nach Gram | Wirksamkeit des Extrakts^{2,}³ |
|---|---|---|
| *Escherichia coli* | Negativ | + |
| *Pseudomonas aeruginosa* | Negativ | + |
| | | |
| *Staphylococcus aureus* | Positiv | ++ |
| *Staphylococcus epidermidis* | Positiv | ++ |

| | | |
|---|---|---|
| ¹ Die getesteten Bakterienstämme sind klinische Isolate, die aus infizierten Patienten stammen. | | |
| ² Getestet wurden jeweils 10 µl Schneckenextrakt, der auf eine Agarplatte aufgebracht wurde, welche vorher mit einer Suspension des jeweiligen Bakteriums beimpft worden war. | | |
| ³ Die Symbole bedeuten folgende Hemmhof-Durchmesser: + 0,9 - 1,2 cm; ++ 1,3 -1,6 cm. | | |

Unter Zugrundelegung eines Molekulargewichts von 60 000 für das wirksame Protein bedeutet dies, daß die Applikation von 460 Femtomol (4,6 x 10⁻¹³ mol) der Substanz ausreicht, um *in vitro* eine vollständige Hemmung des Bakterienwachstums (Durchmesser des Hemmhofs: 1,4 cm) zu erzielen.

### Beispiel 4: Bestimmung der antimikrobiellen Wirkung II

### Material und Methoden:

Das in Beispiel 1 isolierte antibiotisch wirksame Protein wurde in einer Pufferlösung (10 mM TRIS, 100 mM NaCl, pH 9,5) zur Verfügung gestellt. Der Proteingehalt der Lösung betrug 1000 µg/ml.

Zur Prüfung der antimikrobiellen Aktivität des Proteins wurden Bestimmungen der minimalen Hemmkonzentration (MHK) für *Staphylococcus aureus* (einschließlich MRSA), *Enterobacteriaceae* (einschließlich Cefotaxim resistenter Stämme), Non-Fermenter (insbesondere *Pseudomonas aeruginosa*) und *Candida albicans* mittels Mikrodilutionstechnik nach DIN 58940 ausgeführt. Hierzu wurde die gepufferte Proteinlösung in einer geometrischen Verdünnungsreihe mit dem Faktor 2 in sterile Mueller-Hinton Bouillon (pH 7,4) gegeben. Der geprüfte Konzentrationsbereich betrug 0.03 bis 32 mg/l. Nach ca. 20stündiger Inkubation bei 37 °C wurden die MHK-Werte abgelesen.

Zur Qualitätskontrolle wurden ATCC-Referenzstämme mitgeführt: *S. aureus* ATCC 25923, *S. aureus* ATCC 29213, *E. coli* ATCC 25922, *E. coli* ATCC 35218 und *P. aeruginosa* ATCC 27853.

### Ergebnisse:

Insgesamt wurden 77 Isolate aus humanmedizinischen Untersuchungsproben untersucht: 45 *Staphylococcus aureus*, davon 33 methicillinresistent bzw. multiresistent (MRSA); 14 Non-Fermenter, davon 12 *Pseudomonas aeruginosa*; 13 *Enterobacteriaceae,* davon 9 *Escherichia coli*, z. T. cefotaximresistent und 5 *Candida albicans*. Alle MHK-Werte waren gut ablesbar, d. h., es fanden sich scharfe Endpunkte.

Die einzelnen MHK-Werte sind in den Tabellen 4a und 4b aufgelistet. Eine Zusammenfassung der Ergebnisse findet sich in Tabelle 5.
1. Die MHK-Werte für alle *Staphylococcus-aureus-*Isolate liegen zwischen 0,125 und 0,5 mg/l, wobei methicillinresistente Isolate (MRSA) eher eine höhere Sensibilität aufweisen als methicillinsensible (MSSA).
2. Die MHK-Werte für *Pseudomonas aeruginosa* liegen zwischen 2 und ≥ 64 mg/l, meist bei 4 mg/l. Für je einen Stamm von *Pseudomonas species* und *Acinetobacter baumannii* wurden niedrigere MHK-Werte ermittelt.
3. Die MHK-Werte für *Escherichia coli* (Cefotaxim-sensibel oder - resistent) sowie für ein Isolat von *Enterobacter aerogenes* liegen oberhalb des Meßbereichs (≥ 64 mg/l). Für *Citrobacter freundii* (je 1 Cefotaxim-sensibles und -resistentes Isolat) sowie ein Isolat von *Klebsiella pneumoniae* betragen die MHK-Werte 4 bzw. 8 mg/l.
4. Die MHK-Werte für *Candida albicans* liegen in allen untersuchten Fällen oberhalb des Meßbereichs (≥ 64 mg/l).

### Beurteilung der Ergebnisse:

Die MHK-Werte des im Beispiel 1 isolierten Proteins für *Staphylococcus aureus* liegen im Bereich anderer Staphylokokken-spezifischer Antibiotika wie z. B. Vancomycin oder Mupirocin (Pseudomonassäure A).

**Tabelle 4a:**

| Einzelwerte der MHK-Bestimmungen des Peptids von Beispiel 1 | | | |
|---|---|---|---|
| Nr. | Spezies | Identitätsnr. | MHK (mg/l) |
| 1. | *S. aureus* | 29096894 | 0,25 |
| 2. | *S. aureus* | 29096979 | 0,25 |
| 3. | *S. aureus* | 29186557 | 0,25 |
| 4. | *S. aureus* | 290096544 | 0,25 |
| 5. | *S. aureus* | 290096573 | 0,5 |
| 6. | *S. aureus* | 291085630 | 0,25 |
| 7. | *S. aureus* | 291086167 | 0,5 |
| 8. | *S. aureus* | 293114034 | 0,25 |
| 9. | *S. aureus* | ATCC 25923 | 0,5 |
| 10. | *S. aureus* | ATCC 25923 | 0,125 |
| 11. | *S. aureus* | ATCC 29213 | 0,25 |
| 12. | *S. aureus* | ATCC 29213 | 0,25 |
| 13. | *S. aureus* (MRSA) | 29096530 | 0,5 |
| 14. | *S. aureus* (MRSA) | 29096578 | 0,25 |
| 15. | *S. aureus* (MRSA) | 29185647 | 0,25 |
| 16. | *S. aureus* (MRSA) | 29186257 | 0,125 |
| 17. | *S. aureus* (MRSA) | 290064272 | 0,125 |
| 18. | *S. aureus* (MRSA) | 290065119 | 0,125 |
| 19. | *S. aureus* (MRSA) | 290084217 | 0,25 |
| 20. | *S. aureus* (MRSA) | 290085795 | 0,125 |
| 21. | *S. aureus* (MRSA) | 291025969 | 0,125 |
| 22. | *S. aureus* (MRSA) | 291025990 | 0,125 |
| 23. | *S. aureus* (MRSA) | 291034340 | 0,25 |
| 24. | *S. aureus* (MRSA) | 291051784 | 0,125 |
| 25. | *S. aureus* (MRSA) | 291067083 | 0,125 |
| 26. | *S. aureus* (MRSA) | 292055432 | 0,25 |
| 27. | *S. aureus* (MRSA) | 292061054 | 0,25 |
| 28. | *S. aureus* (MRSA) | 292065865 | 0,125 |
| 29. | *S. aureus* (MRSA) | 292078001 | 0,5 |
| 30. | *S. aureus* (MRSA) | 293029965 | 0,25 |
| 31. | *S. aureus* (MRSA) | 293036524 | 0,5 |
| 32. | *S. aureus* (MRSA) | 293036526 | 0,125 |
| 33. | *S. aureus* (MRSA) | 293036839 | 0,25 |
| 34. | *S. aureus* (MRSA) | 293066533 | 0,125 |
| 35. | *S. aureus* (MRSA) | 293067107 | 0,125 |
| 36. | *S. aureus* (MRSA) | 293070769 | 0,125 |
| 37. | *S. aureus* (MRSA) | 293073166 | 0,125 |
| 38. | *S. aureus* (MRSA) | 293074729 | 0,125 |
| 39. | *S. aureus* (MRSA) | 293078957 | 0,125 |
| 40. | *S. aureus* (MRSA) | 293082503 | 0,125 |
| 41. | *S. aureus* (MRSA) | 293087915 | 0,125 |
| 42. | *S. aureus* (MRSA) | 293094792 | 0,125 |
| 43. | *S. aureus* (MRSA) | 293114029 | 0,5 |
| 44. | *S. aureus* (MRSA) | 294044109 | 0,25 |
| 45. | *S. aureus* (MRSA) | 294051030 | 0,125 |

**Tabelle 4b:**

| Einzelwerte der MHK-Bestimmungen des Peptids aus Beispiel 1 | | | |
|---|---|---|---|
| Nr. | Spezies | Identitätsnr. | MHK (mg/l) |
| 46. | *E. coli* | 29096769 | ≥ 64 |
| 47. | *E. coli* | 293115050 | ≥ 64 |
| 48. | *E. coli* | 293116474 | ≥ 64 |
| 49. | *E. coli* | ATCC 25922 | ≥ 64 |
| 50. | *E. coli* | ATCC 35218 | ≥ 64 |
| 51. | *E. coli Cefotaxim Res.* | U-97-10432 | ≥ 64 |
| 52. | *E. coli Cefotaxim Res.* | U-97-11088 | ≥ 64 |
| 53. | *E. coli Cefotaxim Res.* | U-97-15538 | ≥ 64 |
| 54. | *E. coli Cefotaxim Res.* | U-97-9689 | ≥ 64 |
| 55. | *E. aerogenes* | V2-92-33405 | ≥ 64 |
| 56. | *C. freundii* | U-93-13949 | 4 |
| 57. | *C. freundii Cefotaxim Res.* | V1-97-10468 | 4 |
| 58. | *K. pneumoniae* | U-93-13367 | 8 |
| 59. | *P. aeruginosa* | 291087178 | 2 |
| 60. | *P. aeruginosa* | 291087279 | 16 |
| 61. | *P. aeruginosa* | 292084709 | 4 |
| 62. | *P. aeruginosa* | 292084851 | 4 |
| 63. | *P. aeruginosa* | 292084924 | ≥ 64 |
| 64. | *P. aeruginosa* | 293115624 | 8 |
| 65. | *P. aeruginosa* | 293115861 | 2 |
| 66. | *P. aeruginosa* | 293116074 | 4 |
| 67. | *P. aeruginosa* | 294082478 | 4 |
| 68. | *P. aeruginosa* | ATCC 27853 | 8 |
| 69. | *P. aeruginosa* | ATCC 27853 | 4 |
| 70. | *P. aeruginosa* | U-93-11944 | 32 |
| 71. | *Pseudomonas spp.* | U-98-5688 | 0,125 |
| 72. | *A. baumannii* | 29097133 | 2 |
| 73. | *C. albicans* | 291086916 | ≥ 64 |
| 74. | *C. albicans* | 291086918 | ≥ 64 |
| 75. | *C. albicans* | 291087192 | ≥ 64 |
| 76. | *C. albicans* | 292084887 | ≥ 64 |
| 77. | *C. albicans* | 294081971 | ≥ 64 |

**Tabelle 5:**

| Zusammenfassung der Ergebnisse von MHK-Bestimmungen für das in Beispiel 1 isolierte Protein | | | | | | | | | | | | |
|---|---|---|---|---|---|---|---|---|---|---|---|---|
| | n | 0,06 | 0,12 | 0,25 | 0,5 | 1 | 2 | 4 | 8 | 16 | 32 | ≥ 64 |
| *S. aureus* (MSSA) | 12 | 0 | 1 | 8 | 3 | 0 | 0 | 0 | 0 | 0 | 0 | 0 |
| *S. aureus* (MRSA) | 33 | 0 | 20 | 9 | 4 | 0 | 0 | 0 | 0 | 0 | 0 | 0 |
| *P. aeruginosa* | 12 | 0 | 0 | 0 | 0 | 0 | 2 | 5 | 2 | 1 | 1 | 1 |
| *Pseudomonas spp.* | 1 | | 1 | | | | | | | | | |
| *A. baumannii* | 1 | 0 | 0 | 0 | 0 | 0 | 1 | 0 | 0 | 0 | 0 | 0 |
| *E. coli* | 9 | 0 | 0 | 0 | 0 | 0 | 0 | 0 | 0 | 0 | 0 | 9 |
| *C. freundii* | 2 | 0 | 0 | 0 | 0 | 0 | 0 | 2 | 0 | 0 | 0 | 0 |
| *E. aerogenes* | 1 | 0 | 0 | 0 | 0 | 0 | 0 | 0 | 0 | 0 | 0 | 1 |
| *K. pneumoniae* | 1 | 0 | 0 | 0 | 0 | 0 | 0 | 0 | 1 | 0 | 0 | 0 |
| *C. albicans* | 5 | 0 | 0 | 0 | 0 | 0 | 0 | 0 | 0 | 0 | 0 | 5 |

### Beispiel 5: Bestimmung der Wirksamkeit gegen Malaria

Zur Ermittlung der Wirksamkeit der Substanz gegen Malaria wurden 20 Labormäusen nach Standardverfahren mit *Plasmodium bergi* infiziert. Die infizierten Mäuse erhielten eine einmalige Dosis der antibiotisch wirksamen Substanz (50 mg/kg, nicht aufgereinigt,) per intraperitonealer Injektion verabreicht. Die mittlere Überlebensdauer der infizierten Mäuse wurde ermittelt im Vergleich zu einer unbehandelten Kontrollgruppe. Die durchschnittliche Lebensdauer der malariainfizierten Mäuse beträgt ohne Behandlung mit der antibiotisch wirksamen Substanz 4 Tage. Die durchschnittliche Lebensdauer der mit der antibiotisch wirksamen Substanz behandelten Mäuse beträgt 6 Tage.

## Patentansprüche

1. Peptide, Proteine oder Derivate derselben mit antibiotischer Wirkung, die aus dem Schleim der Fußsohle von *Archachatina marginata* isolierbar sind und die mindestens eine der in SEQ ID NOs: 1 bis 8 gezeigten Sequenzen aufweisen, wobei das Peptid- oder Proteinderivat eine glykosylierte, ribosyllerte, acylierte, phosphorylierte, alkoxylierte und/oder amidierte Form des Peptids oder des Proteins ist.

2. Peptide, Proteine oder Derivate derselben nach Anspruch 1, die ein Molekulargewicht von größer als 50 kDa aufweisen.

3. Peptide, Proteine oder Derivate derselben nach Anspruch 1 oder 2, die alle acht, der in SEQ ID NOs: 1 bis 8 gezeigten Sequenzen aufweisen.

4. Peptide, Proteine oder Derivate derselben nach einem oder mehreren der Ansprüche 1 bis 3, die durch rekombinante Techniken erhältlich sind.

5. DNA-Sequenz, die für ein Peptid, Protein oder Derivat derselben nach einem oder mehreren der Ansprüche 1 bis 4 codiert.

6. Vektor, umfassend die DNA-Sequenz nach Anspruch 5.

7. Wirtszelle mit einem Vektor nach Anspruch 6 transformiert ist und/oder die eine DNA-Sequenz gemäß Anspruch 5 aufweist.

8. Verfahren zur Herstellung des in Ansprüchen 1 bis 4 definierten Proteins oder Proteinderivats, umfassend das Kultivieren einer Wirtszelle nach Anspruch 7 und Isolieren des Proteins oder Proteinderivats.

9. Arzneimittel, enthaltend ein Peptid, Protein oder ein Derivat derselben nach einem oder mehreren der Ansprüche 1 bis 4 und gegebenenfalls ein pharmazeutisch akzeptables Trägermaterial.

10. Verwendung eines Peptids, Proteins oder eines Derivats derselben nach einem oder mehreren der Ansprüche 1 bis 4 zur Herstellung eines Medikaments zur Bekämpfung von infektiösen Erregern bei Mensch und Tier.

11. Verwendung nach Anspruch 10, wobei die infektiöse Erreger Bakterien, Viren oder Parasiten sind.

12. Verwendung nach Anspruch 10, wobei die Verbindung geeignet ist, oral oder oral in fraktionierten Präparationen verabreicht zu werden.

13. Verwendung nach Anspruch 10, wobei die Verbindung geeignet ist, intravenös oder durch Hautauftragung verabreicht zu werden.

14. Nahrungs- oder Futtermittel, enthaltend ein Peptid, Protein oder ein Derivat derselben gemäß einem oder mehreren der Ansprüche 1 bis 4.

## Claims

1. Peptides, proteins or derivatives thereof having antibiotic activity, which can be isolated from the mucus of the foot sole of *Archachatina marginata* and which have at least one of the sequences shown in SEQ ID NOs: 1 to 8, wherein said peptide derivative or protein derivative is a glycosylated, ribosylated, acylated, phosphorylated, alkoxylated and/or amidated form of said peptide or protein.

2. The peptides, proteins or derivatives thereof according to claim 1 having a molecular weight of more than 50 kDa.

3. The peptides, proteins or derivatives thereof according to claim 1 or 2 having all eight of the sequences shown in SEQ ID NOs: 1 to 8.

4. The peptides, proteins or derivatives thereof according to one or more of claims 1 to 3 which are obtainable by recombinant techniques.

5. A DNA sequence coding for a peptide, protein or derivative thereof according to one or more of claims 1 to 4.

6. A vector comprising the DNA sequence according to claim 5.

7. A host cell transformed with a vector according to claim 6 and/or having a DNA sequence according to claim 5.

8. A process for the preparation of the protein or protein derivative defined in claims 1 to 4, comprising culturing a host cell according to claim 7 and isolating the protein or protein derivative.

9. A pharmaceutical composition containing a peptide, protein or derivative thereof according to one or more of claims 1 to 4 and optionally a pharmaceutically acceptable carrier material.

10. Use of a peptide, protein or derivative thereof according to one or more of claims 1 to 4 for the preparation of a medicament for controlling infectious pathogens in humans and animals.

11. The use according to claim 10, wherein said infectious pathogens are bacteria, viruses or parasites.

12. The use according to claim 10, wherein the compound is suitable for oral administration or for oral administration in fractionated preparations.

13. The use according to claim 10, wherein the compound is suitable for intravenous administration or for administration by application to the skin.

14. A foodstuff or feed containing a peptide, protein or derivative thereof according to one or more of claims 1 to 4.

## Revendications

1. Peptides, protéines ou dérivés de ceux-ci avec un effet antibiotique, qui sont isolables depuis la matière mucilagineuse de la sole pédieuse d'Archachatina marginata, et qui présentent au moins l'une des séquences montrées dans les SEQ ID Nos : 1 à 8, le dérivé de peptide ou de protéine étant une forme glycosylée, ribosylée, acylée, phosphorylée, alcoxylée et/ou amidisée du peptide ou de la protéine.

2. Peptides, protéines ou dérivés de ceux-ci selon la revendication 1, qui présentent un poids moléculaire supérieur à 50 kDa.

3. Peptides, protéines ou dérivés de ceux-ci selon la revendication 1 ou 2, qui présentent l'ensemble des huit séquences montrées dans les SEQ ID Nos : 1 à 8.

4. Peptides, protéines ou dérivés de ceux-ci selon l'une quelconque ou plusieurs des revendications 1 à 3, qui sont obtenus par des techniques recombinantes.

5. Séquence d'ADN qui code pour un peptide, une protéine ou un dérivé de ceux-ci selon l'une quelconque ou plusieurs des revendications 1 à 4.

6. Vecteur comprenant la séquence d'ADN selon la revendication 5.

7. Cellule hôte transformée par un vecteur selon la revendication 6 et/ou qui présente une séquence d'ADN selon la revendication 5.

8. Procédé destiné à la préparation de la protéine ou du dérivé de protéine défini selon l'une quelconque des revendications 1 à 4, comprenant la culture d'une cellule hôte selon la revendication 7 et l'isolement de la protéine ou du dérivé de protéine.

9. Médicament contenant un peptide, une protéine ou un dérivé de ceux-ci selon l'une quelconque ou plusieurs des revendications 1 à 4 et, le cas échéant, un matériau de support pharmaceutiquement acceptable.

10. Utilisation d'un peptide, d'une protéine ou d'un dérivé de ceux-ci selon l'une quelconque ou plusieurs des revendications 1 à 4 pour la préparation d'un médicament destiné à lutter contre des agents pathogènes infectieux chez l'homme et l'animal.

11. Utilisation selon la revendication 10, dans laquelle les agents pathogènes infectieux sont des bactéries, des virus ou des parasites.

12. Utilisation selon la revendication 10, dans laquelle le composé convient à une administration par voie orale ou par voie orale dans des préparations fractionnées.

13. Utilisation selon la revendication 10, dans laquelle le composé convient à une administration par voie intraveineuse ou par une application cutanée.

14. Aliment pour l'homme ou l'animal contenant un peptide, une protéine ou un dérivé de ceux-ci selon l'une quelconque ou plusieurs des revendications 1 à 4.


### SEQUENZPROTOKOLL

<110> Studiengesellschaft Kohle mbH
<120> Verbindungen mit antibiotischer Wirkung
<130> 001079wo/JH/ml
<140>
   <141>
<160> 9
<170> PatentIn Ver. 2.1
<210> 1
   <211> 26
   <212> PRT
   <213> Archachatina marginata
<400> 1
   Thr Asp Thr Gly Leu Tyr Leu Leu Glu Phe Leu His Thr Phe Thr Glu
   1 5 10 15
   Asp Gly Ser Ile Gln Glu Thr Gly Ile Lys
   20 25
<210> 2
   <211> 19
   <212> PRT
   <213> Archachatina marginata
<400> 2
   Val Ile Leu Ala Ile Pro Gln Ser Ala Leu Ile Glu Leu Asp Trp Lys
   1 5 10 15
   Pro Leu Arg
<210> 3
   <211> 20
   <212> PRT
   <213> Archachatina marginata
<400> 3
   Gln Glu Asn Leu Asn Ala Gln Gly Glu Pro Ile Pro Gly Ser Ala Pro
   1 5 10 15
   Gly Ala Asn Arg
   20
<210> 4
   <211> 14
   <212> PRT
   <213> Archachatina marginata
<400> 4
   Ala Gly Tyr His Cys Gln Tyr Ile Ile Tyr Ile Ile Gln Arg
   1 5 10
<210> 5
   <211> 12
   <212> PRT
   <213> Archachatina marginata
<400> 5
   Gln Glu Gln Asn Gly Asn Leu Met Tyr Leu Asn Arg
   1 5 10
<210> 6
   <211> 13
   <212> PRT
   <213> Archachatina marginata
<400> 6
   Asp Asn Thr Phe Pro Thr Thr Tyr Val Ala Glu Glu Lys
   1 5 10
<210> 7
   <211> 15
   <212> PRT
   <213> Archachatina marginata
<400> 7
   Ile Phe Asp Asp Lys Pro Ile Pro Val Ala Gln Asp Glu Thr Lys
   1 5 10 15
<210> 8
   <211> 17
   <212> PRT
   <213> Archachatina marginata
<400> 8
   Thr Asn Ala Glu Tyr Glu Phe Leu Thr Thr Phe Arg Leu Asn Ala Tyr
   1 5 10 15
   Lys
<210> 9
   <211> 531
   <212> PRT
   <213> Achatina fulica
<400> 9
   Met Leu Leu Leu Asn Ser Ala Leu Phe Ile Leu Cys Leu Val Cys Trp
   1 5 10 15
   Leu Pro Gly Thr Ser Ser Ser Arg Val Leu Thr Arg Arg Glu Gly Pro
   20 25 30
   Gln Cys Ser Arg Ser Val Asp Val Ala Val Val Gly Ala Gly Pro Ser
   35 40 45
   Gly Thr Tyr Ser Ala Tyr Lys Leu Arg Asn Lys Gly Gln Thr Val Glu
   50 55 60
   Leu Phe Glu Tyr Ser Asn Arg Ile Gly Gly Arg Leu Phe Thr Thr His
   65 70 75 80
   Leu Pro Asn Val Pro Asp Leu Asn Leu Glu Ser Gly Gly Met Arg Tyr
   85 90 95
   Phe Lys Asn His His Lys Ile Phe Gly Val Leu Val Lys Glu Leu Asn
   100 105 110
   Leu Ser Asn Lys Glu Phe Thr Glu Gly Phe Gly Lys Pro Gly Arg Thr
   115 120 125
   Arg Phe Phe Ala Arg Gly Lys Ser Leu Thr Leu Glu Glu Met Thr Ser
   130 135 140
   Gly Asp Val Pro Tyr Asn Leu Ser Thr Glu Glu Lys Ala Asn Gln Ala
   145 150 155 160
   Asn Leu Ala Gly Tyr Tyr Leu Lys Lys Leu Thr Gly Phe Asp Gly Glu
   165 170 175
   Val Leu Thr Ile Pro Gln Ala Asn Lys Leu Glu Val Asp Asp Gly Arg
   180 185 190
   Lys Leu Tyr Gln Leu Thr Val Asp Glu Ala Leu Asp Lys Val Gly Thr
   195 200 205
   Pro Glu Gly Lys Glu Phe Leu Lys Ala Phe Ser Thr Gly Asn Thr Glu
   210 215 220
   Phe Ile Glu Gly Val Ser Ala Val Asn Tyr Phe Leu Val Glu Leu Gly
   225 230 235 240
   Glu Arg Glu Glu Glu Ile Leu Thr Leu Thr Asp Gly Met Ser Ala Leu
   245 250 255
   Pro Gln Ala Leu Ala Asp Ala Phe Leu Lys Ser Ser Thr Ser His Ala
   260 265 270
   Leu Thr Leu Asn Arg Lys Leu Gln Ser Leu Ser Lys Thr Asp Asn Gly
   275 280 285
   Leu Tyr Leu Leu Glu Phe Leu Glu Thr Asn Thr His Glu Gly Tyr Thr
   290 295 300
   Glu Glu Ser Asn Ile Thr Asp Leu Val Cys Ala Arg Lys Val Ile Leu
   305                 310                 315                 320
   Ala Ile Pro Gln Ser Ala Leu Ile His Leu Asp Trp Lys Pro Leu Arg
                   325                 330                 335
   Ser Glu Thr Val Asn Glu Ala Phe Asn Ala Val Lys Phe Ile Pro Thr
               340                 345                 350
   Ser Lys Val Phe Leu Thr Phe Pro Thr Ala Trp Trp Leu Ser Asp Ala
           355                 360                 365
   Val Lys Asn Pro Ala Phe Val Val Lys Ser Thr Ser Pro Phe Asn Gln
       370                 375                 380
   Met Tyr Asp Trp Lys Ser Ser Asn Val Thr Gly Asp Ala Ala Met Ile
   385                 390                 395                 400
   Ala Ser Tyr Ala Asp Thr Ser Asp Thr Lys Phe Gln Glu Asn Leu Asn
                   405                 410                 415
   Ser Lys Gly Glu Leu Ile Pro Gly Ser Ala Pro Gly Ala Asn Arg Val
               420                 425                 430
   Thr Val Ala Leu Lys Glu Glu Leu Leu Ser Gln Leu Ser Gln Ala Tyr
           435                 440                 445
   Gly Ile Glu Arg Ser Asp Ile Pro Glu Pro Lys Ser Gly Thr Ser Gln
       450                 455                 460
   Phe Trp Ser Ser Tyr Pro Phe Glu Gly Asp Trp Thr Val Trp Lys Ala
   465                 470                 475                 480
   Gly Tyr His Cys Glu Tyr Thr Gln Tyr Ile Ile Glu Arg Pro Ser Leu
                   485                 490                 495
   Ile Asp Asp Val Phe Val Val Gly Ser Asp His Val Asn Cys Ile Glu
               500                 505                 510
   Asn Ala Trp Thr Glu Ser Ala Phe Leu Ser Val Glu Asn Val Phe Glu
           515                 520                 525
   Lys Tyr Phe
       530